# FASCICULE DE BREVET EUROPEEN

(11) **EP 1 732 525 B1**
(45) Date de publication et mention de la délivrance du brevet: **26.08.2009**
(21) Numéro de dépôt: 05744610.6
(22) Date de dépôt: 23.03.2005
(51) Int. Cl.: A61K 9/70, A61M 35/00, A61K 31/537, A61P 31/10

(54) **PATCH D'AMOROLFINE POUR LE TRAITEMENT DE L'ONYCHOMYCOSE**
AMOROLFIN-PFLASTER ZUR BEHANDLUNG VON ONYCHOMYKOSE
AMOROLFINE PATCH FOR TREATING ONYCHOMYCOSIS

(30) Priorité: 29.03.2004 FR 0403206
(43) Date de publication de la demande: 20.12.2006
(73) Titulaire: GALDERMA RESEARCH & DEVELOPMENT, 06410 Biot (FR)
(72) Inventeur: ZANUTTO, Leslie, P-43230 Paulhaguet (FR); FREDON, Laurent Chemin du Camouyer, F-06330 Roquefort les Pins (FR); MALLARD, Claire, F-06250 Mougins le Haut (FR)
(74) Mandataire: Allab, Myriam
(86) Numéro de dépôt international: PCT/FR2005/000704
(87) Numéro de publication internationale: WO 2005/092299

(56) Documents cités:
- WO-A-01/15670
- WO-A-01/49283
- WO-A-96/19186
- WO-A-99/40955
- WO-A-03/020248
- WO-A-03/020250
- WO-A-20/04000291
- WO-A-20/04021968

## Description

L'invention concerne un dispositif transunguéal comprenant de l'amorolfine ou ses dérivés, notamment son dérivé chlorhydrate, utile dans le traitement de l'onychomycose.

L'onychomycose est une infection mycosique de l'ongle, qui se traduit par des ongles opaques, blancs, épais, friables et fragiles. Elle affecte généralement plus d'un seul ongle. L'onychomycose atteint 2 à 13 % de la population et augmente à environ 15-20 % chez les personnes âgées de 40 à 60 ans.

Les traitements de l'onychomycose couramment utilisés se classent en trois catégories :
- les traitements systémiques et locaux d'antifongiques ;
- les interventions chirurgicales pour enlever l'ongle infecté en tout ou partie suivies d'un traitement topique pour les tissus exposés ; ou bien
- les applications topiques de crèmes, lotions, gels ou solutions sur l'ongle infecté.

Ces différentes approches présentent de multiples inconvénients.

L'administration systémique (par voie orale) d'agents antifongiques pour le traitement de l'onychomycose ne donne un effet thérapeutique qu'à long terme. Par exemple, le traitement par voie orale avec le composé antifongique Kétoconazole nécessite typiquement l'administration de 200 à 400 mg par jour pendant six mois avant qu'un bénéfice thérapeutique significatif soit obtenu. Or, ces agents antifongiques peuvent à long terme provoquer des effets secondaires indésirables non négligeables.

L'ablation de l'ongle par voie chirurgicale comporte également un certain nombre d'inconvénients, dont notamment la douleur et l'inconfort associés à l'intervention chirurgicale et l'apparence inesthétique de l'ongle.

Dans le cas de traitements par voie topique habituels à l'aide de crèmes, lotions, gels ou solutions, la diffusion du principe actif à travers la surface de l'ongle est très faible et la durée du traitement est particulièrement longue. De plus, ces formes de dosage topique ne permettent pas de maintenir le principe actif en contact avec l'ongle pendant une durée prolongée et des bandages doivent donc être utilisées.

Une autre forme topique connue est le vernis à ongle (Murdan et al., International Journal of Pharmaceutics, 236 (2002), 1-26). Le Locéryl® est un exemple de vernis composé d'amorolfine (5 %), d'Eudragit RL 100, de triacétate de glycérole, d'acétate de butyle, d'acétate d'éthyle et d'éthanol. Le vernis est appliqué sur la plaque cornée de l'ongle et séché pendant quelques minutes pour évaporer les solvants et laisser un film de polymère imperméable à l'eau sur la surface de l'ongle. Le principe actif est ensuite libéré du film et diffuse à travers la plaque cornée de l'ongle.

Le Locéryl® est appliqué une à deux fois par semaine pendant six mois sur les ongles des mains et neuf à douze mois pour les ongles des pieds. La durée de traitement dépend essentiellement de l'intensité, de la localisation de l'infection et de la surface de l'ongle atteinte.

Le traitement des ongles par un vernis s'avère ainsi très contraignant car il est répétitif, demande un entretien de l'ongle avant chaque application et exige une attention particulière afin d'éviter toute contamination des ongles non atteints.

La demande WO 96/19186 décrit un pansement comprenant une couche adhésive en forme de T, une compresse (pad) flexible comprenant une cavité, ladite cavité ayant la forme d'un ongle et contenant un moyen d'absorption (couche poreuse) contenant de l'urée et un composé thiol tel que représenté à la page 7 de ladite demande WO 96/19186 (i.e., formule générale (I)).

La couche adhésive de cette demande ne contient pas de principe actif (agent antifongique), ni de promoteur d'absorption.

La demande WO 2004/021968 divulgue l'utilisation d'un mélange d'agents pro-pénétrants agissant en synergie, choisis parmi l'urée, un acide, et de l'ethoxydiglycol pour la préparation d'une solution à application unguéale ou péri-unguéale.

La demande WO 99/40955 décrit de son côté un patch transdermique et transunguéal pour traiter l'onychomycose comprenant un agent antifongique, tel que le fluconazole, la terbinafine, le clotrimazole, le miconazole et le kétoconazole ou leurs sels, mais ne cite pas le chlorhydrate d'amorolfine.

Les inventeurs ont maintenant mis en évidence que l'amorolfine, notamment sous forme chlorhydrate, est capable de diffuser efficacement à travers la plaque cornée unguéale à l'aide d'un dispositif matriciel bioadhésif transunguéal. Un tel dispositif transunguéal est destiné à être appliqué exclusivement sur la surface de l'ongle malade.

L'invention a ainsi pour objet un dispositif transunguéal comprenant :
- un support de couchage non occlusif; et
- une couche adhésive matricielle comprenant a) un principe actif présent en quantité entre 20% et 40% en poids de la couche et choisi parmi l'amorolfine et ses dérivés, solubilisé ou dispersé dans un adhésif et b) soit un promoteur d'absorption unique qui est l'exaltolide, soit au moins deux promoteurs d'absorption choisis parmi le groupe constitué par l'urée, la N-acétylcystéine et l'acide lactique.

Dans ce qui suit, sauf indication contraire, les proportions des différents constituants de la couche adhésive matricielle sont exprimées en pourcentage en poids (m/m) de masse sèche de la couche matricielle adhésive.

De préférence, l'amorolfine ou ses dérivés est présente en une quantité comprise entre 20 et 40% en poids de la couche adhésive matricielle. De façon préférée, l'adhésif est un adhésif sensible à la pression et est de préférence choisi parmi les polyacrylates, les polyisobutylènes, les silicones ou un mélange de ceux-ci.

L'adhésif peut représenter 10 à 98 % en poids de la couche adhésive matricielle et mieux encore de 25 à 85 % en poids.

De façon privilégiée, le support de couchage comprend un polymère choisi parmi le polyuréthane, le polyéthylène, le poly(éthylène-co-acétate de vinyle), le chlorure de polyvinyle ou un mélange de ceux-ci.

Le dispositif transunguéal selon la présente invention est anhydre. Par anhydre, on entend un dispositif contenant moins de 2,5% d'eau.

La quantité de chaque promoteur d'absorption est de préférence comprise entre 1 à 30 % en poids de la couche matricielle adhésive, mieux encore de 2,5 à 15 % en poids.

Selon un mode préféré, la couche matricielle adhésive contient deux promoteurs d'adsorption choisis parmi l'urée, la N-acétylcystéine et l'acide lactique.

Selon un mode de réalisation préféré, le promoteur d'absorption est l'urée associée à l'acide lactique ou à la N-acétylcystéine.

Selon un premier mode de réalisation, le promoteur d'absorption est le mélange urée / acide lactique, chacun représentant indépendamment, de préférence, de 2 à 6 % en poids de la couche matricielle adhésive.

Selon un second mode de réalisation, le promoteur d'absorption est le mélange urée / N-acétylcystéine, chacune représentant indépendamment de 2 à 10 % en poids de la couche matricielle adhésive.

Selon un autre mode de réalisation, le promoteur d'absorption est unique et correspond à l'exaltolide seul, qui représente de préférence 10 à 20 % en poids de la couche matricielle adhésive.

Selon un mode préférentiel, le dispositif comprend de plus un revêtement extérieur amovible qui est déposé sur la couche matricielle adhésive.

Selon un deuxième aspect, l'invention a pour objet un procédé de préparation d'un dispositif selon l'invention comprenant les étapes de :
i) préparation d'un mélange comprenant (1) l'adhésif, (2) soit un unique promoteur d'absorption qui est l'exaltolide, soit au moins deux promoteurs d'absorption choisis parmi le groupe constitué par l'urée, la N-acétylcystéine et l'acide lactique, et (3) un principe actif choisi parmi l'amorolfine et ses dérivés, dans un solvant, ledit adhésif étant miscible audit solvant ;
ii) couchage du mélange obtenu sur le support de couchage ; et
iii) évaporation du solvant.

Selon un mode préférentiel, l'adhésif est un polyacrylate. Dans ce cas, le solvant est de préférence l'éthanol.

Selon un autre mode de réalisation préféré, l'adhésif est un silicone. Dans ce cas, le solvant est de préférence le dichlorométhane.

Selon un mode de réalisation, l'amorolfine ou ses dérivés est solubilisée dans le mélange obtenu en i).

Par forme solubilisée, on entend une dispersion à l'état moléculaire dans un liquide, aucune cristallisation de l'actif n'étant visible à l'oeil nu ni même au microscope optique en polarisation croisée.

Selon un autre mode de réalisation, l'amorolfine ou ses dérivés est dispersée dans le mélange obtenu en i).

De préférence, le mélange obtenu en i) comprend deux promoteurs d'absorption de l'amorolfine dans l'ongle. Alternativement, le mélange obtenu en i) comprend préférentiellement l'exaltolide comme unique promoteur d'absorption de l'amorolfine dans l'ongle. De façon particulière, le mélange obtenu en i) comprend de plus un co-solvant capable de solubiliser l'amorolfine ou ses dérivés et/ou le(s) promoteur(s) d'adsorption dans le mélange.

Le co-solvant est de préférence l'isopropanol.

Le solvant et, le cas échéant, le co-solvant peuvent représenter de 2 à 90 % en poids du mélange obtenu à l'étape i).

De façon privilégiée, le procédé comprend une étape iv) de mise en place du revêtement extérieur amovible.

Selon un troisième aspect, l'invention a pour objet l'utilisation d'amorolfine ou ses dérivés pour la fabrication d'un médicament destiné au traitement de l'onychomycose, ledit médicament étant sous la forme d'un dispositif transunguéal selon l'invention.

Selon un quatrième aspect, l'invention a pour objet l'utilisation d'amorolfine ou ses dérivés pour la fabrication d'un médicament destiné au traitement de l'onychomycose, ledit médicament étant sous la forme d'un dispositif transunguéal obtenu par le procédé selon l'invention.

Selon un premier aspect, l'invention a pour objet un dispositif transunguéal comprenant :
- un support de couchage non occlusif; et
- une couche adhésive matricielle comprenant a) de l'amorolfine ou un de ses dérivés présent en quantité entre 20% et 40% en poids de la couche et solubilisée ou dispersée dans un adhésif et b) soit un promoteur d'absorption unique qui est l'exaltolide, soit au moins deux promoteurs d'absorption choisis parmi le groupe constitué par l'urée, la N-acétylcystéine et l'acide lactique.

Par amorolfine, on entend l'amorolfine base. Par dérivés d'amorolfine, on entend notamment ses sels pharmaceutiquement acceptables, et plus particulièrement son dérivé chlorhydrate, appelé ci-après amorolfine HCl.

Par sels pharmaceutiquement acceptables, on entend des sels compatibles avec la peau, les muqueuses et/ou les phanères.
L'amorolfine HCl ou chlorhydrate d'amorolfine est un dérivé hydrochloré de l'amorolfine et désigne le chlorhydrate du cis-4-[3-[4-(1,1-diméthyl-propyl)phényl]-2-méthylpropyl]-2,6-diméthyl-morpholine.

Ce composé chimique exerce une activité fongistatique et fongicide par inhibition de la synthèse des stérols de la membrane cellulaire des champignons tels que les levures, les dermatophytes, les moisissures et les dematiées (champignons noirs).

Par dispositif « transunguéal », on entend un dispositif rigide ou souple destiné à être appliqué exclusivement sur l'ongle et à y adhérer, permettant d'obtenir la libération et la pénétration d'un principe actif sur une zone unguéale ciblée. Ce dispositif se distingue d'un dispositif transdermique car il ne déborde en aucun cas de la surface de l'ongle ; il ne couvre pas les parties de peau adjacentes, éliminant ainsi les risques de passage systémique.

De manière simplifiée, on utilisera indifféremment les expressions « dispositif transunguéal » et « patch ».

Les patchs de l'invention peuvent présenter diverses structures.

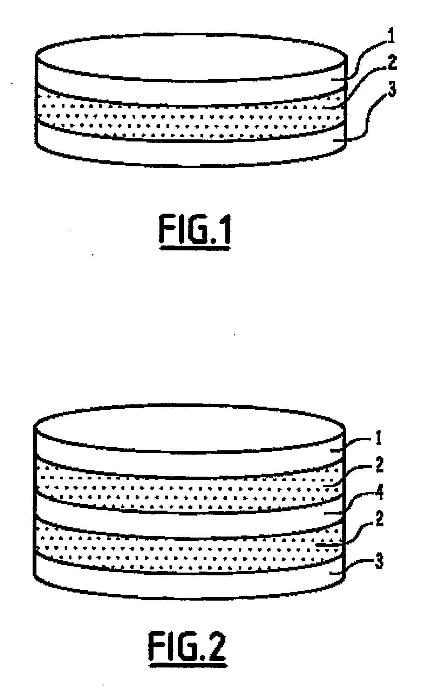

A titre illustratif, un dispositif matriciel mono-couche et un dispositif matriciel multi-couches sont respectivement représentés à la figure 1 et à la figure 2.

Le dispositif matriciel mono-couche se compose d'un support de couchage (1), d'une couche adhésive matricielle unique (2) et du revêtement extérieur amovible (3).

De son côté, le dispositif matriciel multi-couches se compose d'au moins deux couches matricielles, dont l'une au moins des deux couches est une couche adhésive. Chacune est séparée par une membrane (4), laquelle permet une libération progressive de l'amorolfine ou un de ses dérivés, éventuellement en combinaison avec d'autres principes actifs.

### La couche de support de couchage

Le support de couchage du dispositif transunguéal est la partie du patch visible lorsqu'il est appliqué sur la peau.

Il permet de maintenir le principe actif au contact de l'ongle pour accroître la diffusion du principe actif à l'intérieur de l'ongle.

Il peut être non occlusif.

Le support de couchage permet également de protéger la couche en contact avec l'ongle de toute contamination.

Pour des raisons esthétiques et de discrétion, le support de couchage sera de préférence incolore ou de couleur.

Il présentera de préférence une flexibilité suffisante pour s'adapter au mieux à la surface de l'ongle même dans le cas de surface irrégulière.

Il se compose de polymères choisis principalement de manière à présenter une inertie et une résistance chimique envers les composants de la formule du patch, à savoir notamment l'amorolfine ou un de ses dérivés, l'adhésif et les solvants éventuels, ainsi que le(s) promoteur(s) d'absorption.

Son choix peut être en outre fonction de l'apparence, et de la nécessité ou non de flexibilité. A cette fin, la résistance à l'élongation et la perméabilité à la vapeur d'eau et d'oxygène des polymères pourront être prises en considération.

De façon préférée, le support de couchage comprend un polymère choisi parmi le polyuréthane, le polyéthylène, le poly(éthylène-co-acétate de vinyle), le chlorure de polyvinyle ou un mélange de ceux-ci.

Comme exemples de support de couchage pouvant convenir à la réalisation d'un patch selon l'invention, on peut citer les composés commercialisés sous les références *Dow BLF 2015, Dow BLF 2023, Dow BLF 2050, Dow BLF 2052, Dow BLF 2057* et *Dow BLF 2080* par Dow Chemicals.

### La couche adhésive matricielle

Par « matrice » ou « couche adhésive matricielle », on entend au sens de la présente invention un mélange d'au moins un principe actif dont l'amorolfine ou un de ses dérivés, à l'état dispersé ou solubilisé, de façon homogène, dans un adhésif biocompatible et sensible à la pression, mélange qui contient en outre soit l'exaltolide soit au moins deux promoteurs d'absorption de l'amorolfine ou un de ses dérivés solubilisés ou dispersés de façon homogène.

Dans le cas d'un dispositif mono-couche, cette couche matricielle possède une surface directement en contact avec le support de couchage et, une deuxième surface directement en contact avec le revêtement extérieur amovible à ôter lors de l'application du patch sur la zone à traiter.

Lors de l'utilisation, ce revêtement extérieur amovible est retiré de ladite surface de la couche matricielle qui est alors appliquée directement sur l'ongle.

Le principe actif et les promoteurs d'absorption diffusent à travers l'adhésif et ses mélanges le cas échéant, jusqu'à la surface de l'ongle.

Le principe actif et les promoteurs d'absorption diffusent à travers l'adhésif et ses mélanges le cas échéant, jusqu'à la surface de l'ongle.

Cette couche matricielle constitue donc une forme de dosage unitaire d'une composition d'amorolfine ou un de ses dérivés sur un support adhésif contenant soit l'exaltolide soit au moins deux promoteurs d'absorption du principe actif dans l'ongle.

### l'adhésif

L'adhésif au sens de la présente description, désigne un polymère ou mélange de polymères chimiquement inertes vis-à-vis des différents composants de la matrice dont notamment l'amorolfine ou un de ses dérivés et les promoteurs d'absorption.

Il a pour principale fonction d'assurer l'adhésion du patch sur l'ongle.

Il est avantageusement choisi de telle sorte qu'il ne laisse aucun résidu sur l'ongle lors du retrait du patch.

Il présente de préférence une bonne adhérence vis-à-vis du support de couchage.

Les adhésifs utilisables selon l'invention sont des adhésifs sensibles à la pression (« Pressure Sensitive Adhesive ou PSA»). Parmi les adhésifs PSA pouvant convenir à la réalisation d'un patch selon l'invention, on peut distinguer principalement les deux classes suivantes :
- Les adhésifs sans solvant, appelés « Hot Melt ». Ils se caractérisent par un état solide aux températures inférieures à 180°F et présentent de faibles viscosités au dessus de 180°F. Ils sont par conséquent formulables à chaud. Parmi les adhésifs sans solvant, on peut citer différents copolymères comme notamment les copolymères de vinyle acétate d'éthylène, les copolymères styrène-isopropène-styrène, les copolymères styrène-butadiène-styrène les polyuréthane réactifs (PUR). Généralement ces polymères ne présentent pas à eux seuls les caractéristiques de performance requises pour la réalisation d'un produit fini. Par conséquent, ce type d'adhésif sans solvant « Hot Melt » sera de préférence utilisé en présence de cires, d'antioxydants, de plastifiants ou autres additifs dont le but sera d'améliorer les performances de l'adhésif.
- Les adhésifs avec solvant, appelés « Solvent-based adhesives ». Ces adhésifs en solution présentent de bonnes propriétés d'adhésion utilisés seuls et ne nécessitent pas par conséquent de plastifiants ou autres additifs. De plus, ces composés sont formulables à froid.

Par conséquent, compte tenu de leurs propriétés définies ci-dessus, les adhésifs « Solvent based adhesives » sont les adhésifs préférentiellement utilisés pour la réalisation d'un patch selon l'invention.

Parmi les adhésifs « Solvent based adhesives » pouvant convenir à la réalisation d'un patch selon l'invention, on préfère particulièrement ceux choisis parmi les polyacrylates, les polyisobutylènes, les silicones ou un mélange de ceux-ci.

Par « polyacrylate » ou « acrylique », on entend un polymère de formule : 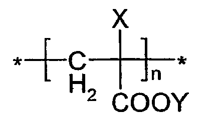 dans laquelle
X représente de préférence un atome d'hydrogène ou un alkyle en C₁-C₁₂ ;
Y représente de préférence un atome d'hydrogène ou un alkyle en C₁-C₁₂ éventuellement substitué ;
n représente le nombre d'unités monomères.

Par « polyisobutylène », on entend de préférence un polymère de formule : 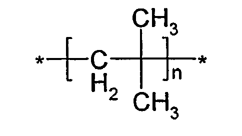 dans laquelle n représente le nombre d'unités monomères.

Par «silicone », on entend un polymère polysiloxane de formule : (RR'SiO)n, dans laquelle R et R' sont identiques ou différents et représentent de préférence un atome d'hydrogène ou un groupe alkyle en C₁-C₁₂.

« Alkyle » désigne un groupe hydrocarboné aliphatique qui peut être linéaire ou ramifié ayant 1 à 12 atomes de carbone, de préférence 1 à 6 atomes de carbone dans la chaîne. Ramifié signifie qu'un ou plusieurs groupes alkyles inférieurs, tels que le méthyle, l'éthyle ou le propyle, sont liés à une chaîne alkyle linéaire.

« Alkyle inférieur » signifie 1 à 4 atomes de carbone dans la chaîne qui peut être linéaire ou ramifiée.

L'alkyle peut être substitué par un ou plusieurs « substituants de groupe alkyle », qui peuvent être identiques ou différents et comprennent les groupes hydroxyle, amino, alkylamino ou dialkylamino.

A titre d'exemples d'adhésifs acryliques, on peut notamment citer les polymères acryliques vinyle acétate commercialisés en solution par la société National Starch, sous la dénomination Duro-Tak^{®} 387-2287, Duro-Tak^{®} 387-2516, et les polymères acryliques commercialisés en solution sous la dénomination Duro-Tak^{®} 387-2353.

A titre d'exemples de polyisobutylènes, on peut notamment citer les composés de type polymère hydrocarboné paraffinique, composés de macromolécules à longues chaînes linéaires se terminant par des liaisons oléfiniques. Ces composés sont commercialisés sous le nom de Vistanex^{®} LM MS LL et Vistanex^{®} MM L80, lesquels se présentent sous forme solide.

A titre d'exemple de silicones, on peut citer notamment les « Pressure Sensitive Adhesives» tels que les tétra (triméthylsiloxy) silane et notamment les composés commercialisés par Dow Corning sous le nom de BIO PSA^{®}, particulièrement la série des BIO PSA 4200^{®} et plus particulièrement les adhésifs BIO PSA 7-4102^{®}, BIO PSA 7 -4202^{®} et du BIO PSA 7-4302^{®}. Ces trois composés se présentent sous forme de solution et diffèrent par leurs propriétés adhésives.

Au sein de la couche matricielle, l'adhésif est présent en une quantité allant de préférence de 10 à 98 % en poids de la couche matricielle adhésive et plus préférentiellement de 25 à 85% en poids.

### L'amorolfine ou ses dérivés

L'amorolfine ou un de ses dérivés peut être à l'état solubilisé ou à l'état dispersé dans la couche matricielle adhésive.

Dans tous les cas, il est souhaitable que l'amorolfine ou un de ses dérivés soit uniformément répartie au sein de la couche matricielle adhésive.

Mieux encore, elle possède une granulométrie homogène.

De préférence, l'amorolfine ou un de ses dérivés est présente dans la couche matricielle adhésive en une quantité comprise entre 20 et 40 % en poids de la couche matricielle adhésive.

De préférence, on utilise l'amorolfine HCl dans les compositions selon la présente invention.

L'amorolfine ou un de ses dérivés peut être présente en combinaison avec une autre substance active adaptée à une application transunguéale possédant des propriétés biologiques ou pharmacologiques, notamment antibiotique ou antifongique, par application topique sur la zone de l'ongle à traiter.

### Les promoteurs d'absorption dans l'ongle

La couche matricielle adhésive comprend en outre soit l'exaltolide, soit au moins deux promoteurs d'absorption de l'amorolfine ou ses dérivés dans l'ongle.

On entend par « promoteur d'absorption dans l'ongle » des composés chimiques pharmaceutiquement acceptables capables d'accroître la perméabilité d'une membrane biologique telle que la peau ou l'ongle vis-à-vis de l'amorolfine ou ses dérivés, de manière à augmenter la cinétique de pénétration de l'amorolfine ou ses dérivés à travers la membrane.

Cette cinétique de pénétration peut être mesurée en utilisant un appareillage de diffusion cellulaire tel que les cellules de Franz décrites par Merrit et al. (Diffusion Apparatus for Skin Pénétration, J. Controlled Release, 1984, 1, 161-162).

Les promoteurs d'absorption sont bien connus de l'état de la technique et peuvent inclure notamment des α-hydroxyacides, des esters d'acides gras et des amides de ceux-ci, des alcools gras, des acides gras et des esters de glycérol notamment le 2-(2-éthoxyéthoxy)-éthanol, le glycérolmonolaurate, le propylène glycol, les polyéthylène glycols, les glycérides polyglycosylés, les polyglycols insaturés (Labrafil M1 944CS^{®},Gattefosse), les polyglycérides saturés (Labrasol, Gattefosse), Labrafac HydroWL1219^{®} (Gattefosse), le décylméthylsulfoxide, les pyrrolidones, l'acide salicylique, l'acide lactique, le myristate d'isopropyle, le diméthylformamide, le diméthylacétamide, le dodécylsulfate de sodium, les phospholipides, Transcutol^{®} (Gattefosse), des mélanges d'acide oléique et de 2-(2-éthoxyéthoxy)-éthanol, d'acide oléique et Labrafil^{®}, ces mélanges d'acide oléique préférentiellement dans un rapport d'approximativement 1:1. Des composés enzymatiques, tel que des enzymes protéolytiques qui facilitent la pénétration des principes actifs à travers les tissus kératiniques ou à travers l'ongle, peuvent également être utilisés comme promoteurs d'absorption. A titre d'exemples non limitatifs d'acides gras utilisables selon l'invention, on peut citer, les acides caprique, laurique, myristique, palmitique, stéarique, arachidique, béhénique, lignocérique, myristoléique, palmitoléique, pétrosélinique, oléique, linoléique et linolénique.

D'autres promoteurs d'absorption connus fonctionnent par le biais d'une hydrolyse, kératolyse, dénaturation ou autre mécanisme équivalent qui détruisent l'ongle ou la membrane. A titre d'exemples de promoteurs d'absorption fonctionnant ainsi, on peut citer l'urée, les acides aminés comportant des groupes sulfydryles, les alkylsulfoxides, et tout composé équivalent qui fonctionne en détruisant ou dénaturant l'ongle et /ou la membrane permettant ainsi au composé pharmaceutique de pénétrer les couches profondes de la membrane.

A titre d'exemple de promoteurs d'absorption, on peut citer notamment l'urée, l'exaltolide, la N-acétylcystéine et l'acide lactique ou un mélange de ceux-ci, l'urée associée à l'acide lactique ou à la N-acétylcystéine, et l'exaltolide seul, étant particulièrement préférés.

De préférence, les promoteurs d'absorption sont solubilisés, et ce de façon homogène dans la couche matricielle adhésive.

La quantité de chaque promoteur d'absorption représente entre 0,1 à 30 % en poids de la couche matricielle adhésive, plus préférentiellement de 1 à 20 % en poids et mieux encore de 2 à 10 % en poids.

De façon plus spécifique, lorsque le promoteur d'absorption est l'urée en combinaison avec l'acide lactique, il est particulièrement préféré que l'urée et l'acide lactique représentent chacun indépendamment 2 à 6 % en poids de la couche matricielle adhésive.

Lorsque le promoteur d'absorption est l'urée en combinaison avec la N-acétylcystéine, il est particulièrement préféré que l'urée et la N-acétylcystéine représentent chacune indépendamment de 2 à 10 % en poids de la couche matricielle adhésive, et mieux encore qu'elles soient présentes à raison de 5 à 10 % pour l'urée et 2 à 6 % pour la N-acétylcystéine en poids de la couche matricielle adhésive.

### Les additifs

La couche matricielle adhésive contenue dans le dispositif transunguéal utilisable selon l'invention est caractérisée par le fait qu'elle ne comprend aucun additif, ce dernier ayant été éliminé par évaporation.

En effet, lors de la fabrication du dispositif transunguéal selon l'invention, l'utilisation de solvants et /ou co-solvants organiques ou aqueux est nécessaire pour l'homogénéisation du mélange comprenant l'adhésif, au moins deux promoteurs d'adsorption et l'amorolfine ou un de ses dérivés. De tels solvants et/ou co-solvants sont ensuite éliminés de la couche matricielle adhésive par évaporation.

De tels solvants et/ou co-solvants sont les seuls additifs utilisables selon la présente invention. Ils sont présents dans la couche matricielle adhésive uniquement pendant sa fabrication.

### Le revêtement extérieur amovible

Le dispositif transunguéal comprend généralement un revêtement extérieur amovible.

Par revêtement extérieur amovible, on entend un support protecteur en contact direct avec la couche matricielle adhésive qui est enlevé juste avant l'application sur la peau. Il doit donc être inerte chimiquement et imperméable vis-à-vis de la formule du patch.

A titre d'exemple de matériau pouvant convenir à titre de revêtement extérieur amovible, on peut citer notamment une feuille d'aluminium, un papier contenant une feuille de polyéthylène ou un polyester siliconé.

### Membranes séparatrices

Le patch peut comprendre en outre une ou plusieurs membranes ou couches séparatrices : il s'agit alors notamment d'un dispositif matriciel multi-couches tel que représenté à la figure 2.

Ces membranes séparatrices sont formées de polymères inertes chimiquement tels que le polyéthylène ou le polypropylène.

Ces membranes peuvent être poreuses ou non poreuses.

Dans le cas des membranes poreuses, l'amorolfine ou ses dérivés se libèrent par diffusion directe à travers la membrane.

Dans le cas des membranes non poreuses, la vitesse de passage de l'amorolfine ou ses dérivés dépend de sa solubilité dans la membrane et de l'épaisseur de celle-ci.

Les patchs selon l'invention peuvent être préparés selon des techniques conventionnelles telles que l'enduction dite « hot melt », c'est-à-dire en l'absence de solvant, ou « en phase solvante ».

Selon un deuxième aspect, l'invention a pour objet un procédé de préparation d'un dispositif transunguéal comprenant les étapes de :
i) préparation d'un mélange comprenant (1) l'adhésif, (2) soit l'exaltolide, soit au moins deux promoteurs d'absorption choisis dans le groupe constitué par l'acide lactique, la N-acétylcystéine et l'urée, et (3) l'amorolfine ou un de ses dérivés, dans un solvant, ledit adhésif étant miscible audit solvant ;
ii) couchage du mélange obtenu sur le support de couchage ; et
iii) évaporation du solvant.

Ce procédé est basé sur le principe de la technique classique d'enduction en phase solvante.

### Etape (i) de préparation du mélange adhésif

Le choix du solvant est essentiellement déterminé par la nature de l'adhésif.

Il est choisi en fonction de sa capacité à solubiliser l'adhésif et de sa volatilité. En effet, une volatilité trop importante rend difficile l'obtention d'un couchage uniforme.

A cette fin, le solvant peut être par exemple choisi parmi les esters carboxyliques, les hydrocarbures halogénés, les hydrocarbures aliphatiques, les éthers, les cétones, les solvants polaires non protiques et les alcools, en particulier les alcanols.

Comme exemple d'esters carboxyliques, on peut mentionner en particulier l'acétate de méthyle, l'acétate d'éthyle, l'acétate d'isopropyle, l'acétate de butyle, l'acétate d'amyle, et le propionate d'éthyle.

Des exemples d'hydrocarbures halogénés sont notamment le chloroforme, dichlorométhane, et 1,2-dichloroéthane.

Des exemples d'hydrocarbures aliphatiques englobent en particulier le pentane, hexane, heptane, et octane.

Des exemples d'éthers sont notamment le diéthyl éther, le dibutyl éther, le dioxane, et le tétrahydrofurane.

Comme exemple de cétones, on peut mentionner l'acétone et la méthyléthylcétone.

Comme exemple de solvants polaires non protiques, on peut citer l'acétonitrile, le N,N-diméthylformamide, et le diméthylsulfoxyde.

Selon un mode de réalisation préféré, l'adhésif mis en oeuvre à l'étape i) est un polyacrylate. Dans ce cas, sont préférés à titre de solvant les alcools, mieux encore l'éthanol.

Selon un autre mode de réalisation préféré, l'adhésif mis en oeuvre à l'étape i) est un silicone. Dans ce cas, sont préférés à titre de solvant, les hydrocarbures halogénés, tels que notamment le dichlorométhane.

Le choix du solvant peut aussi dépendre de l'état dans lequel on souhaite obtenir l'amorolfine ou ses dérivés, à savoir solubilisé ou dispersé.

Ainsi, le cas échéant, lorsque plusieurs solvants sont possibles, ce solvant peut être choisi de façon à solubiliser également les autres constituants de la couche matricielle tels que l'amorolfine ou ses dérivés et/ou le(s) promoteur(s) d'absorption.

Toutefois, l'utilisation d'un co-solvant ou d'un mélange de co-solvants peut dans certains cas, s'avérer nécessaire.

Le choix du co-solvant dépend de la nature de la solution d'adhésif ainsi que de la nature du constituant que l'on souhaite solubiliser.

Le co-solvant doit être compatible avec la solution d'adhésif, notamment être miscible vis-à-vis du solvant et de l'adhésif, et éventuellement avec les autres constituants.

Il peut être notamment choisi parmi les hydrocarbures halogénés tels que le dichlorométhane, les alcools tels que l'éthanol ou l'isopropanol ou un mélange de ceux-ci.

En particulier, l'amorolfine HCl étant particulièrement soluble dans l'éthanol, celui-ci peut être utile à titre de co-solvant avec un solvant autre que l'éthanol.

Dans le cas des adhésifs acryliques, l'éthanol peut donc avantageusement permettre de solubiliser à la fois l'adhésif et l'amorolfine HCl, sans qu'il soit nécessaire d'ajouter un co-solvant pour solubiliser celle-ci.

A titre d'exemple, l'éthanol ou l'isopropanol, seuls ou en combinaison avec le dichlorométhane, sont particulièrement utiles à titre de co-solvant pour solubiliser le couple promoteur d'absorption urée / acide lactique, notamment avec les adhésifs silicones. L'isopropanol est dans ce but particulièrement préféré.

Bien entendu, l'homme du métier pourra, à la lumière de ses connaissances générales, déterminer quels sont les co-solvants les plus appropriés aux conditions mises en oeuvre et à l'état solubilisé ou dispersé des constituants recherché.

L'ensemble des solvants et co-solvant sera présent dans des proportions allant de 2 à 90 % en poids, de préférence entre 10 et 75 % en poids du mélange obtenu à l'étape (i) et plus préférentiellement entre 20 et 70 %.

### Etape (ii) de couchage

La solution ou la suspension obtenue à l'étape i), plus ou moins visqueuse, est enduite sur le support de couchage.

Cette étape peut être réalisée avec un applicateur de film fourni dans le commerce.

### Etape (iii) d'évaporation du solvant

Le solvant est évaporé soit à l'air libre, soit à la chaleur au moyen d'un socle chauffant ou d'une étuve par exemple.

Selon un troisième aspect, l'invention a pour objet l'utilisation d'amorolfine ou un de ses dérivés pour la fabrication d'un médicament destiné au traitement de l'onychomycose, ledit médicament étant sous la forme d'un dispositif transunguéal tel que défini dans la présente description.

Selon un quatrième aspect, l'invention a pour objet l'utilisation d'amorolfine ou un de ses dérivés pour la fabrication d'un médicament destiné au traitement de l'onychomycose, ledit médicament étant sous la forme d'un dispositif transunguéal obtenu par le procédé tel que défini dans la présente description.

Tous les types d'onychomycose sont visés, à savoir notamment l'onychomycose distal-subunguéale, l'onychomycose proximal-subunguéale blanche, l'onychomycose blanche superficielle et l'onychomycose induite par une souche du genre *Candida.*

Le patch d'amorolfine ou un de ses dérivés selon l'invention est une forme d'administration qui offre de nombreux avantages dans le traitement des onychomycoses, notamment en ce qu'il permet un traitement local, une application simple et rapide.

Il a également pour avantage d'augmenter le temps de contact entre l'ongle et le principe actif sans que des manipulations soient nécessaires et de libérer le principe actif en continu sur une longue durée.

Les patchs selon la présente invention présentent en outre l'avantage d'éviter toute étape de prétraitement de l'ongle avant application, et notamment de prétraitement par abrasion.

Selon un autre mode préféré selon l'invention, le patch peut être utilisé en combinaison avec un traitement systémique pour le traitement de l'ongle. Une combinaison du patch selon l'invention avec un traitement systémique a notamment été prouvée comme efficace dans le traitement des pathologies de l'ongle, et plus particulièrement dans le traitement de l'onychomycose.

Le patch selon l'invention peut être fabriqué dans toute taille et forme convenant à l'utilisation requise, tel que des formes circulaires, ovales rectangulaires ou carrées. De préférence, le patch selon l'invention aura une forme la mieux adaptée à la forme de l'ongle, à savoir de type ovale. La taille préférée du patch selon l'invention est située entre 0,5 cm² et 4 cm² et choisie en fonction de la localisation de la zone à traiter.

Un autre avantage du patch selon l'invention est de pouvoir être mis en place sur l'ongle pour une durée d'une semaine sans avoir à être changé tous les jours. Ainsi, un excès d'amorolfine ou ses dérivés est de préférence utilisé pour assurer que la quantité d'actif au sein du patch sera présente en quantité suffisante après une semaine d'application.

Les exemples et figures illustrent l'invention sans en limiter la portée.

### FIGURES

La figure 1 est une représentation schématique d'un dispositif matriciel mono-couche.
La figure 2 est une représentation schématique d'un dispositif matriciel multi-couches.
La figure 3 représente, pour différentes formulations de patch, une évaluation de la libération de l'amorolfine HCl, exprimée en terme de masse des zones d'inhibition.

### EXEMPLES

### • Matériel et méthodes

### Les couches de support de couchage

Les supports de couchage sous la dénomination Dow BLF 2023® et Dow BLF 2080® mis en oeuvre sont commercialisés par Dow Chemicals.

### Les adhésifs

Le polymère acrylique utilisé est un polymère en solution dans l'acétate d'éthyle principalement commercialisé sous le nom de Duro-Tak® 387-2516 par National Starch.

Le polymère silicone BIO PSA 7-4102^{®} est un polymère en solution dans l'acétate d'éthyle fourni par Dow Corning.

### Le revêtement extérieur amovible

Le revêtement extérieur amovible 3M 9956^{®} utilisé est un film de polyester fourni par la société 3M. Le côté en contact avec le substrat est traité par un polymère fluoré. Il est compatible avec les adhésifs utilisés. Il est transparent, sensible à la chaleur et son épaisseur est de 74 ± 5 µm.

### Mode opératoire général de préparation des patchs

Les patchs ont été préparés par la technique d'enduction en phase solvante comprenant :
- le mélange de tous les composants de la couche matricielle adhésive dans le solvant, l'adhésif devant être impérativement miscible dans ce solvant pour assurer une bonne homogénéité du couchage ; le mélange est agité à l'aide d'un agitateur magnétique jusqu'à l'obtention d'une préparation homogène ;
- environ 30 g de préparation sont prélevés afin de réaliser l'enduction de la solution obtenue, plus ou moins visqueuse, sur le support de couchage;
- enfin, l'évaporation du solvant, à l'air libre sous hotte pendant 24 heures à température ambiante.

Les pourcentages m/m représentent les pourcentages des différents constituants exprimés par rapport à la masse sèche de la couche matricielle après évaporation des solvants.

Les couchages ont été réalisés avec un applicateur de Film KCC 101 fourni par la société Erichsen avec les barres de couchage à fil spiralé de 400 µm ou de 100 µm d'épaisseur.

Dans les tableaux ci-après, les pourcentages en poids correspondent au poids des différents constituants de la solution destinée à former la couche adhésive matricielle après évaporation des solvants.

### Analyse des patchs

L'éventuelle formation de cristaux d'amorolfine HCl et l'homogénéité du couchage des patchs sont observés au microscope avec un grossissement variant de x50 à x250 et les photographies sont prises grâce au logiciel d'acquisition d'images « Kappa ».

### Mode opératoire pour les tests d'évaluation microbiologiques

Ce test permet de déterminer l'efficacité des formulations réalisées. L'expérience s'est effectuée sur des onglons de porcs qui présentent une physiologie proche de ceux des humains.

### ➢ Principe de la manipulation

Les patchs sont déposés sur des onglons de porc préalablement réhydratés. Ces onglons sont déposés sur une gélose Sabouraud (référence AEB152352 d'AES laboratoire) infectée par un inoculum de spores de *Trichophyton rubrum,* impliqué dans les onychomycoses. Les géloses et onglons sont alors placés au réfrigérateur à +4°C pendant un temps déterminé, afin de permettre la diffusion du principe actif à travers l'onglon puis la gélose. Les géloses sont ensuite placées dans un incubateur pendant 48 heures à +32,5°C, température idéale pour la prolifération du *Trichophyton rubrum.*

La souche ne pousse pas en présence de l'amorolfine HCl. Des zones d'inhibition sur la gélose peuvent donc être observées, ce qui permet d'évaluer la libération de l'amorolfine HCl.

### ➢ Déroulement de la manipulation

La manipulation se déroule, en conditions stériles, en plusieurs étapes :
Réhydratation des onglons de porcs,
Préparation des géloses infectées,
Applications des patchs sur les onglons de porc,
Mise au réfrigérateur à +4°C pendant un temps défini,
Incubation à +32,5°C pendant 48 heures,
Lecture des résultats

### ➢ Réhydratation des onglons de porc

Préalablement, les onglons de porc sont grattés afin d'éliminer toute la chair, puis soumis à l'autoclave. Il est alors nécessaire de les réhydrater avant leur utilisation. Une zone délimitée par une couronne indique une surface sur laquelle est disposée la formule. Cette couronne doit être collée manuellement sur l'onglon avant réhydratation. Ces deux opérations sont effectuées en même temps :
- placer une gaze stérile dans une boite de Pétri ronde,
- humidifier celle-ci avec de l'eau stérilisée,
- découper les rondelles,
- mettre de la colle sur les rondelles,
- prendre un onglon, vérifier son intégrité,
- coller la rondelle sur l'onglon en évitant que la colle déborde à l'intérieur de la rondelle,
- placer l'onglon sur la gaze humide et laisser le temps de préparer la souche.

### ➢ Préparation des gélose infectées

Les différentes étapes sont les suivantes :
- prendre des cultures de travail sur tubes inclinés,
- gratter ces cultures avec du bouillon Sabouraud (référence 111 105 d'AES laboratoire) afin de récupérer les spores,
- filtrer cette suspension pour éliminer tous les résidus de gélose,
- diluer de manière à avoir suffisamment de suspension pour infecter les géloses (soit 1 ml de suspension pour 100 ml de gélose),
- faire une dilution au 10^{e} de la suspension,
- placer la dilution dans la cellule de Thoma
- laisser reposer un quart d'heure,
- compter les spores au microscope,
- calculer le nombre de souches dans la suspension,
- rapporter ce chiffre à la puissance 7 ou 8,
- si la concentration est bonne la manipulation continue.

Dans le cas inverse, diluer la solution de départ ou gratter à nouveau les cultures pour récupérer de nouveaux spores.
- Placer la solution au vortex pour bien homogénéiser,
- prélever la quantité de suspension nécessaire,
- ajouter la suspension à la gélose Sabouraud,
- bien agiter,
- placer les boîtes de manière plane grâce à un niveau et de la pâte à modeler, proche d'un Bec Bunsen,
- couler les boîtes de Pétri en plaçant 250 ml de gélose infectée dans chaque boîte,
- laisser prendre en masse, en laissant les boites entrouvertes,
- fermer les boîtes,
- les placer sous la hotte à flux laminaire,
- ouvrir complètement les boîtes,
- laisser sécher complètement pendant une demi-heure.

### ➢ Applications des patchs

Les étapes de cette manipulation sont les suivantes :
- découper un échantillon grâce à un emporte-pièce de diamètre précis (8 mm),
- le coller sur l'onglon à l'intérieur de la rondelle,
- placer les onglons sur la gélose, quatre onglons par boîte,
- puis placer les boîtes au réfrigérateur (+4°C) pendant un temps déterminé.

### ➢ Diffusion et incubation

La diffusion de l'actif à travers l'onglon et la gélose se fait pendant le passage au réfrigérateur car le trychophyton ne peut pas pousser. L'incubation correspond à la période durant laquelle le champignon peut pousser.

Les dernières étapes sont les suivantes :
- sortir les boîtes du réfrigérateur après le temps prévu,
- enlever les onglons à l'aide d'une pince,
- placer les boîtes à l'étuve (+32,5°C) pour que le Trichophyton rubrum puisse se développer, généralement 48 heures suffisent pour avoir des zones d'inhibitions nettes.

### ➢ Lecture des résultats

Lorsque le champignon a suffisamment poussé, il apparaît des zones d'inhibition traduisant la diffusion du principe actif à travers l'onglon jusqu'à la gélose. Selon cette diffusion, la zone d'inhibition est plus ou moins importante. La confrontation des résultats se fait par comparaison de ces zones. Le protocole opératoire est alors le suivant :
- décalquer les zones d'inhibitions,
- découper et identifier ces zones,
- peser chaque zone.

Chaque formulation est évaluée sur plusieurs points (n=3). Les moyennes des différents résultats sont présentées sous forme de graphique.

### EXEMPLE 1 - Patch acrylique d'amorolfine HCl solubilisée en l'absence de promoteur d'absorption

### Préparation de la couche adhésive matricielle :

| Constituants | % en poids | % (m/m) Masse sèche |
|---|---|---|
| Ethanol absolu | 58,43 | -- |
| Ethyl acétate | 5,40 | -- |
| Butyl acétate | 2,46 | -- |
| Amorolfine HCl | 5,09 | 29,87 |
| Duro-Tak 387-2516 | 28,62 | 70,13 |

La préparation est limpide, un peu liquide. Le polymère acrylique se solubilise bien dans l'éthanol, de même que l'amorolfine HCl.

Les résultats des tests sur les patchs sont les suivants :

| Support de couchage | BLF 2023 | BLF 2080 |
|---|---|---|
| Quantité déposée (g) | 2,42 | 2,95 |
| Collant par rapport au revêtement extérieur amovible | Se décolle bien | Se décolle bien |
| Collant sur l'ongle | Colle bien | Colle bien |
| Epaisseur (µm) | 52 ± 19,4 | 96 ± 40,2 |

### EXEMPLE 2 - Patch acrylique d'amorolfine HCl dispersée en l'absence de promoteur d'absorption

La formule de la couche adhésive matricielle réalisée est donc la suivante :

| Constituants | % en poids | % (m/m) Masse sèche |
|---|---|---|
| Duro-Tak 387-2516 | 84,73 | 69,98 |
| amorolfine HCl | 15,27 | 30,02 |

Les caractéristiques du patch sont les suivantes :

| Support de couchage | BLF 2023 | BLF 2080 |
|---|---|---|
| Quantité déposée (g) | 2,99 | 5,50 |
| Collant par rapport au revêtement extérieur amovible | Se décolle bien | Se décolle bien |
| Collant sur l'ongle | Colle bien | Colle bien |
| Epaisseur (µm) | 203 ± 14,9 | 240 ±11,9 |

Les patchs ont un aspect plus opaque car les cristaux d'amorolfine HCl sont très gros.

Les épaisseurs sont très élevées car les préparations sont très concentrées en amorolfine HCl et en adhésif.

### EXEMPLE 3 - Patch acrylique d'amorolfine HCl solubilisée en présence du promoteur d'absorption Urée/Acide lactique

### Préparation de la couche adhésive matricielle :

| Constituants | % en poids | % (m/m) Masse sèche |
|---|---|---|
| Ethanol absolu | 58,10 | -- |
| Ethyl acétate | 5,28 | -- |
| Butyl acétate | 2,21 | -- |
| Acide lactique | 1,34 | 5,25 |
| Urée | 0,96 | 3,37 |
| amorolfine HCl | 5,20 | 28,74 |
| Duro-Tak 387-2516 | 27,51 | 62,64 |

Les résultats des tests réalisés avec les patchs ainsi formulés sont les suivants :

| Support de couchage | BLF 2023 | BLF 2080 |
|---|---|---|
| Quantité déposée (g) | 2,38 | 1,83 |
| Collant par rapport au revêtement extérieur amovible | Se décolle bien | Se décolle bien |
| Collant sur l'ongle | Colle bien | Colle bien |
| Epaisseur (µm) | 86 ± 45,1 | 89 ± 38,1 |

D'après les photographies microscopiques, avec les deux supports de couchage, l'amorolfine HCl est recristallisée.

Par ailleurs, avec le support de couchage BLF 2023, l'adhésif se décolle particulièrement bien.

### EXEMPLE 4 - Patch acrylique d'amorolfine HCl dispersée en présence du promoteur d'absorption Urée/Acide lactique

### Préparation de la couche adhésive matricielle :

| Constituants | % en poids | % (m/m) Masse sèche |
|---|---|---|
| Ethanol absolu | 9,55 | -- |
| Ethyl acétate | 51.20 | -- |
| Butyl acétate | 2,01 | -- |
| Acide lactique | 0,79 | 4,24 |
| Urée | 0,85 | 3,94 |
| Amorolfine HCl | 5,92 | 29,55 |
| Duro-Tak 387-2516 | 29,68 | 62,27 |

Les résultats des tests réalisés avec les patchs ainsi formulés sont les suivants :

| Support de couchage | BLF 2023 | BLF 2080 |
|---|---|---|
| Quantité déposée (g) | 2,41 | 2,21 |
| Collant par rapport au revêtement extérieur amovible | Se décolle bien | Se décolle bien |
| Collant sur l'ongle | Colle bien | Colle bien |
| Epaisseur (µm) | 60 ± 34,2 | 100 ± 48,2 |

Les patchs présentent de bonnes propriétés adhésives quelque soit le type de support de couchage utilisé.

### EXEMPLE 5 - Patch acrylique d'amorolfine HCl solubilisée en présence du promoteur d'absorption Exaltolide

La formule de la couche adhésive matricielle réalisée est la suivante :

| Constituants | % en poids | % (m/m) Masse sèche |
|---|---|---|
| Ethanol absolu | 56,78 | -- |
| Ethyl acétate | 5,65 | -- |
| Butyl acétate | 2,12 | -- |
| Exaltolide | 2,99 | 14,93 |
| Amorolfine HCl | 5,84 | 29,23 |
| Duro Tak 387-2516 | 26,62 | 55,84 |

Les caractéristiques des patchs ainsi formulés sont les suivantes :

| Support de couchage | BLF 2023 | BLF 2080 |
|---|---|---|
| Quantité déposée (g) | 2,53 | 1,32 |
| Collant par rapport au revêtement extérieur amovible | Se décolle bien | Se décolle bien |
| Collant sur l'ongle | Colle bien | Colle mais il faut bien appuyer |
| Epaisseur (µm) | 86 ± 52,0 | 100 ± 61,4 |

L'exaltolide recristallise quel que soit le support de couchage.

Une recristallisation plus uniforme est observée avec le support de couchage BLF 2080.

Pour cette formulation, de meilleures propriétés adhésives sont observées pour le support de couchage BLF 2023.

### EXEMPLE 6 - Patch acrylique d'amorolfine HCl dispersée en présence d'exaltolide

La formule de la couche adhésive matricielle réalisée est la suivante :

| Constituants | % en poids | % (m/m) Masse sèche |
|---|---|---|
| Ethyl acétate | 25,83 | -- |
| Butyl acétate | 0,87 | -- |
| Exaltolide | 6,21 | 12,40 |
| amorolfine HCl | 12,5 | 25,04 |
| Duro-Tak 387-2516 | 54,59 | 62,56 |

Les caractéristiques des patchs sont les suivantes :

| Support de couchage | BLF 2023 | BLF 2080 |
|---|---|---|
| Quantité déposée (g) | 2,46 | 2,87 |
| Aspect des patchs | Couchage homogène Opalescent | Couchage homogène Opalescent |
| Collant par rapport au revêtement extérieur amovible | Se décolle bien | Se décolle bien |
| Collant sur l'ongle | Colle bien | Colle bien mais en appuyant un peu |
| Epaisseur (µm) | 223 ± 32,4 | 194 ± 47,1 |

Les épaisseurs ne sont pas significativement différentes entre les deux supports de couchages.

Le couchage est homogène et de bonnes propriétés adhésives ont été obtenues.

### EXEMPLE 7 - Patch acrylique d'amorolfine solubilisée en présence de N-acétvl-L-cystéine et d'urée

La formule de la couche adhésive matricielle réalisée est la suivante :

| Constituants | % en poids | % (m/m) Masse sèche |
|---|---|---|
| Ethanol absolu | 48,69 | -- |
| Ethyl acétate | 4,32 | -- |
| Butyl acétate | 2,22 | -- |
| Urée | 2,49 | 9,83 |
| N-acétyl cystéine | 1,25 | 4,93 |
| amorolfine HCl | 7,58 | 29,82 |
| Duro Tak 387-2516 | 33,47 | 55,42 |

Les caractéristiques des patchs ainsi formulés sont les suivantes :

| Support de couchage | BLF 2023 | BLF 2080 |
|---|---|---|
| Quantité déposée | 5,24 g | 3,44 g |
| Collant par rapport au revêtement extérieur amovible | Se décolle bien | Se décolle bien |
| Collant sur l'ongle | Colle bien | Colle bien |
| Epaisseur (µm) | 148 ± 41,8 | 105 ± 31,0 |

Avec le support de couchage BLF 2023, la recristallisation de l'amorolfine HCl est plus uniforme.

L'épaisseur n'est pas identique sur les deux supports de couchages. Le dépôt sur le support de couchage BLF 2023 est nettement plus important que celui sur le support de couchage BLF 2080.

Enfin, il est à noter que le support de couchage BLF 2023 permet un meilleur décollement de l'adhésif.

### EXEMPLE 8 - Patch silicone d'amorolfine HCl dispersée en l'absence de promoteur d'absorption

Les formules réalisées sont les suivantes :

| Constituants | % en poids | % (m/m) Masse sèche |
|---|---|---|
| BIO PSA 7-4302 | 78,42 | 70,23 |
| amorolfine HCl | 21,58 | 29,77 |

Les caractéristiques des patchs sont les suivantes :

| Support de couchage | BLF 2023 | BLF 2080 |
|---|---|---|
| Quantité déposée (g) | 2,33 | 2,71 |
| Collant par rapport au revêtement extérieur amovible | Se décolle bien | Se décolle bien |
| Collant sur l'ongle | Colle bien | Colle bien |
| Epaisseur (µm) | 421 ± 35,3 | 287 ± 35,7 |

Les patchs sont très épais car il y a peu ou pas d'évaporation.

### EXEMPLE 9 - Patch silicone d'amorolfine HCl dispersée en présence d'Urée et d'acide lactique

La formule suivante est réalisée :

| Constituants | % en poids | % (m/m) Masse sèche |
|---|---|---|
| Ethanol absolu | 5.70 | -- |
| Ethyl acétate | 67,58 | -- |
| Butyl acétate | 1.91 | -- |
| Urée | 0.48 | 2,59 |
| Acide lactique | 0.74 | 4,02 |
| amorolfine HCl | 5.38 | 29,15 |
| BIO PSA 7-4302 | 18.21 | 64,24 |

L'éthanol absolu est nécessaire pour solubiliser l'urée.

Les patchs présentent les caractéristiques suivantes :

| Support de couchage | BLF 2023 | BLF 2080 |
|---|---|---|
| Quantité déposée (g) | 3,53 | 3,74 |
| Aspect des patchs Aspect des patchs | Couchage très homogène, très pâle. Il paraît très fin. | Couchage très homogène, très pâle. Il paraît très fin. |
| Collant sur l'ongle | Colle bien | Colle bien |
| Epaisseur (µm) | 33 ± 13,3 | 40 ± 24,1 |

Les patchs sont très fins mais ils restent très homogènes.

### EXEMPLE 10 - Patch silicone d'amorolfine HCl dispersée en présence d'Exaltolide

La formule suivante est réalisée :

| Constituants | % en poids | % (m/m) Masse sèche |
|---|---|---|
| Ethyl acétate | 31,64 | -- |
| Butyl acétate | 1,15 | -- |
| Exaltolide | 7,66 | 14,79 |
| amorolfine HCl | 15,84 | 30,50 |
| BIO PSA 7-4302 | 43.71 | 54,71 |

L'exaltolide est très soluble dans l'acétate d'éthyle. Un solvant supplémentaire n'est pas nécessaire. La teneur finale en exaltolide dans le patch est de 15% (m/m), l'amorolfine HCl de 30%.

Les caractéristiques des patchs sont les suivantes :

| Support de couchage | BLF 2023 | BLF 2080 |
|---|---|---|
| Quantité déposée (g) | 1,67 | 3,74 |
| Collant par rapport au revêtement extérieur amovible | Se décolle mal | Se décolle mal |
| Collant sur l'ongle | Colle bien | Colle bien |
| Décollement de l'ongle | Assez bon | Assez bon |

Le couchage est uniforme.

### EXEMPLE 11 - Evaluation microbiologique des patchs décrits aux exemples 1 à 10

Les conditions opératoires sont identiques pour tous les patchs, 72 heures de diffusion au réfrigérateur et test sur trois points différents avec les onglons laissés sur la gélose lors du passage à l'étuve.

Tableau de résultat :

| Exemple | Masse des zones d'inhibition (moyenne de n=3) en mg | Ecart-type |
|---|---|---|
| Ex 1 | 80 | 23,26 |
| Ex 2 | 106,33 | 15,53 |
| Ex 3 | 192,67 | 2,31 |
| Ex 4 | 186,67 | 49,01 |
| Ex 5 | 81,67 | 7,77 |
| Ex 6 | 193,33 | 51,55 |
| Ex 7 | 167,33 | 39,5 |
| Ex 8 | 86,67 | 23,8 |
| Ex 9 | 202,33 | 91,6 |
| Ex 10 | 128,33 | 27,47 |

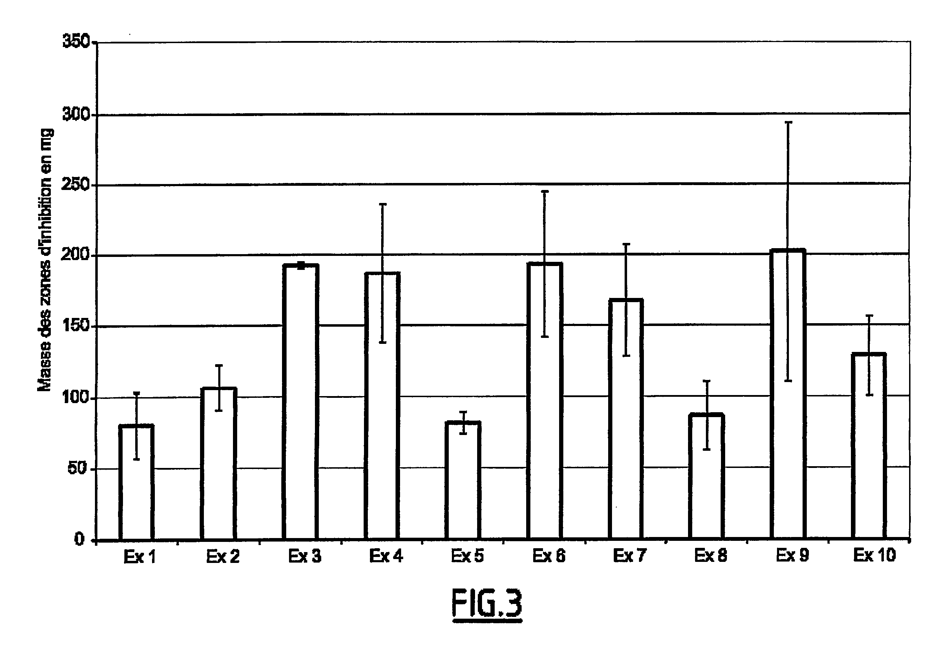

La figure 3 représente le récapitulatif des résultats de tous les tests effectués sur les patchs.

Cette évaluation microbiologique montre la capacité de l'amorolfine HCl à diffuser à partir des patchs différemment formulés. Cette diffusion ne semble pas être dépendante de la nature de l'adhésif (acrylique ou silicone), ni être dépendante de la préparation du principe actif préalablement solubilisé ou dispersé.

En revanche, elle met en évidence l'effet des promoteurs d'absorption sur la diffusion de l'amorolfine HCl à partir des patchs.

Ainsi, les résultats d'efficacité sont globalement homogènes pour l'urée et l'acide lactique avec une augmentation de l'efficacité normalisée de l'ordre de 114 % par rapport aux patchs sans promoteurs d'absorption.

Pour l'exaltolide, les augmentations d'efficacité sont très hétérogènes, allant de 2,5 % pour l'amorolfine HCl solubilisée avec le Duro-Tak^{®} 387-2516 à 121 % pour l'amorolfine HCl dispersée dans le BIO PSA 7-4302^{®}. L'exaltolide apparaît donc comme un promoteur d'absorption un peu moins efficace avec des résultats variables selon la nature de l'adhésif.

Pour le couple urée / N-acétyl-L-cystéine, l'augmentation par rapport à la formulation correspondante sans promoteur est de 108 %. La N-acétyl-L-cystéine constitue donc une alternative à l'acide lactique lorsqu'elle est combinée à l'urée.

## Revendications

1. Dispositif transunguéal comprenant:
- un support de couchage non occlusif; et
- une couche adhésive matricielle comprenant
a) un principe actif présent en quantité entre 20% et 40% en poids de la couche et choisi parmi l'amorolfine et ses dérivés, solubilisé ou dispersé dans un adhésif, et
b) soit l'exaltolide, soit au moins deux promoteurs d'absorption choisis parmi l'urée, la N-acétylcystéine et l'acide lactique.

2. Dispositif selon la revendication 1, **caractérisé en ce que** le principe actif est l'amorolfine HCl.

3. Dispositif selon la revendication 1 ou 2, **caractérisé en ce qu'**il est anhydre.

4. Dispositif selon l'une des revendications 1 à 3, **caractérisé en ce que** la couche matricielle adhésive ne contient aucun additif.

5. Dispositif selon l'une des revendications 1 à 4, dans lequel les promoteurs d'absorption sont l'urée associée à l'acide lactique ou à la N-acétylcystéine.

6. Dispositif selon l'une des revendications 1 à 4, dans lequel le promoteur d'absorption est l'exaltolide seul.

7. Dispositif selon l'une quelconque des revendications précédentes, dans lequel l'adhésif est un adhésif sensible à la pression.

8. Dispositif selon l'une quelconque revendications précédentes, dans lequel l'adhésif est choisi parmi les polyacrylates, les polyisobutylènes, les silicones ou un mélange de ceux-ci.

9. Dispositif selon l'une quelconque des revendications précédentes, dans lequel l'adhésif représente 10 à 98 % en poids de la couche adhésive matricielle, de préférence de 25 à 85 % en poids.

10. Dispositif selon l'une quelconque des revendications précédentes, dans lequel le support de couchage comprend un polymère choisi parmi le polyuréthane, le poly(éthylène-co-acétate de vinyle), le polyéthylène, le chlorure de polyvinyle ou un mélange de ceux-ci.

11. Dispositif selon l'une quelconque des revendications précédentes, dans lequel la quantité de chaque promoteur d'absorption est comprise entre 1 à 30 % en poids de la couche matricielle adhésive, de préférence de 2,5 à 15 % en poids.

12. Dispositif selon la revendication 5, dans lequel l'urée et l'acide lactique représentent chacun indépendamment de 2 à 6 % en poids de la couche matricielle adhésive.

13. Dispositif selon la revendication 5, dans lequel l'urée et la N-acétylcystéine représentent chacune indépendamment de 2 à 10 % en poids de la couche matricielle adhésive.

14. Dispositif selon la revendication 6, dans lequel l'exaltolide représente de 10 à 20 % en poids de la couche matricielle adhésive.

15. Dispositif selon l'une quelconque des revendications précédentes, comprenant de plus un revêtement extérieur amovible, qui est déposé sur la couche matricielle adhésive.

16. Procédé de préparation d'un dispositif selon l'une quelconque des revendications 1 à 15, comprenant les étapes de :
i) préparation d'un mélange comprenant (1) l'adhésif, (2) soit l'exaltolide, soit au moins deux promoteurs d'absorption choisis parmi l'urée, l'acide lactique et la N-acétylcystéine, et (3) l'amorolfine ou un de ses dérivés dans un solvant, ledit adhésif étant miscible audit solvant ;
ii) couchage du mélange obtenu sur le support ; et
iii) évaporation du solvant.

17. Procédé selon la revendication 16, dans lequel l'adhésif est un polyacrylate.

18. Procédé selon la revendication 16, dans lequel l'adhésif est un silicone.

19. Procédé selon la revendication 17, dans lequel le solvant est l'éthanol.

20. Procédé selon la revendication 18, dans lequel le solvant est le dichlorométhane.

21. Procédé selon l'une quelconque des revendications 16 à 20, dans lequel l'amorolfine ou ses dérivés est solubilisée dans le mélange obtenu en i).

22. Procédé selon l'une quelconque des revendications 16 à 20, dans lequel l'amorolfine ou ses dérivés est dispersée dans le mélange obtenu en i).

23. Procédé selon l'une quelconque des revendications 16 à 22, dans lequel le mélange obtenu en i) comprend de plus un co-solvant capable de solubiliser l'amorolfine ou ses dérivés et/ou le(s) promoteur(s) d'absorption, dans le mélange.

24. Procédé selon la revendication 23, dans lequel le co-solvant est l'isopropanol.

25. Procédé selon l'une quelconque des revendications 26 à 24, dans lequel le solvant, et le cas échéant le co-solvant, représentent 2 à 90 % en poids du mélange obtenu à l'étape i).

26. Procédé selon la revendication 16, comprenant en outre une étape iv) de mise en place du revêtement extérieur amovible.

27. Utilisation d'amorolfine ou ses dérivés pour la fabrication d'un médicament destiné au traitement de l'onychomycose, ledit médicament étant sous la forme d'un dispositif transunguéal tel que défini dans l'une quelconque des revendications 1 à 15.

28. Utilisation d'amorolfine ou ses dérivés pour la fabrication d'un médicament destiné au traitement de l'onychomycose, ledit médicament étant sous la forme d'un dispositif transunguéal obtenu par le procédé selon l'une quelconque des revendications 16 à 26.

## Claims

1. Transungual device comprising:
- a nonocclusive coating support; and
- an adhesive matrix layer comprising
a) an active principle present in an amount of between 20% and 40% by weight of the layer and chosen from amorolfine and its derivatives, dissolved or dispersed in an adhesive, and
b) either exaltolide or at least two absorption promoters chosen from urea, N-acetylcysteine and lactic acid.

2. Device according to Claim 1, **characterized in that** the active principle is amorolfine hydrochloride.

3. Device according to Claim 1 or 2, **characterized in that** it is anhydrous.

4. Device according to one of Claims 1 to 3, **characterized in that** the adhesive matrix layer does not comprise any additive.

5. Device according to one of Claims 1 to 4, in which the absorption promoters are urea in combination with lactic acid or with N-acetylcysteine.

6. Device according to one of Claims 1 to 4, in which the absorption promoter is exaltolide alone.

7. Device according to any one of the preceding claims, in which the adhesive is a pressure-sensitive adhesive.

8. Device according to any one of the preceding claims, in which the adhesive is chosen from polyacrylates, polyisobutylenes, silicones or a blend of these.

9. Device according to any one of the preceding claims, in which the adhesive represents from 10 to 98% by weight of the adhesive matrix layer, preferably from 25 to 85% by weight.

10. Device according to any one of the preceding claims, in which the coating support comprises a polymer chosen from polyurethane, poly(ethylene-co-vinyl acetate), polyethylene, polyvinyl chloride or a blend of these.

11. Device according to any one of the preceding claims, in which the amount of each absorption promoter is between 1 and 30% by weight of the adhesive matrix layer, preferably from 2.5 to 15% by weight.

12. Device according to Claim 5, in which the urea and the lactic acid each independently represent from 2 to 6% by weight of the adhesive matrix layer.

13. Device according to Claim 5, in which the urea and the N-acetylcysteine each independently represent from 2 to 10% by weight of the adhesive matrix layer.

14. Device according to Claim 6, in which the exaltolide represents from 10 to 20% by weight of the adhesive matrix layer.

15. Device according to any one of the preceding claims, additionally comprising a removable external coating which is deposited on the adhesive matrix layer.

16. Process for the preparation of a device according to any one of Claims 1 to 15, comprising the stages of:
i) preparing a mixture comprising (1) adhesive, (2) either exaltolide or at least two absorption promoters chosen from urea, lactic acid and N-acetylcysteine, and (3) amorolfine or one of its derivatives, in a solvent, the said adhesive being miscible with the said solvent;
ii) coating the mixture obtained on the support; and
iii) evaporating the solvent.

17. Process according to Claim 16, in which the adhesive is a polyacrylate.

18. Process according to Claim 16, in which the adhesive is a silicone.

19. Process according to Claim 17, in which the solvent is ethanol.

20. Process according to Claim 18, in which the solvent is dichloromethane.

21. Process according to any one of Claims 16 to 20, in which amorolfine or its derivatives is dissolved in the mixture obtained in i).

22. Process according to any one of Claims 16 to 20, in which amorolfine or its derivatives is dispersed in a mixture obtained in i).

23. Process according to any one of Claims 16 to 22, in which the mixture obtained in i) additionally comprises a cosolvent capable of dissolving amorolfine or its derivatives and/or the absorption promoter(s) in the mixture.

24. Process according to Claim 23, in which the cosolvent is isopropanol.

25. Process according to any one of Claims 16 to 24, in which the solvent and, if appropriate, the cosolvent represent from 2 to 90% by weight of the mixture obtained in stage i).

26. Process according to Claim 16, additionally comprising a stage iv) of fitting the removable external coating.

27. Use of amorolfine or its derivatives in the manufacture of a medicament intended for the treatment of onychomycosis, the said medicament being in the form of a transungual device as defined in any one of Claims 1 to 15.

28. Use of amorolfine or its derivatives in the manufacture of a medicament intended for the treatment of onychomycosis, the said medicament being in the form of a transungual device obtained by the process according to any one of Claims 16 to 26.

## Patentansprüche

1. Transunguale Vorrichtung, die umfasst:
- einen nicht okklusiven Beschichtungsträger; und
- eine klebefähige Matrixschicht, die umfasst:
a) einen Wirkstoff, der in einer Menge von 20 bis 40 Gew.-% der Schicht enthalten ist und der unter Amorolfin und seinen Derivate ausgewählt wird, der in einem Klebstoff solubilisiert oder dispergiert ist, und
b) Exaltolid oder mindestens zwei Absorptionsförderer, die unter Harnstoff, N-Acetylcystein und Milchsäure ausgewählt werden.

2. Vorrichtung nach Anspruch 1, **dadurch gekennzeichnet, dass** es sich bei dem Wirkstoff um Amorolfin-HCl handelt.

3. Vorrichtung nach Anspruch 1 oder 2, **dadurch gekennzeichnet, dass** sie wasserfrei ist.

4. Vorrichtung nach einem der Ansprüche 1 bis 3, **dadurch gekennzeichnet, dass** die klebefähige Matrixschicht keinen Hilfsstoff enthält.

5. Vorrichtung nach einem der Ansprüche 1 bis 4, wobei es sich bei den Absorptionsförderern um Harnstoff in Kombination mit Milchsäure oder N-Acetylcystein handelt.

6. Vorrichtung nach einem der Ansprüche 1 bis 4, wobei der Absorptionsförderer ausschließlich aus Exaltolid besteht.

7. Vorrichtung nach einem der vorhergehenden Ansprüche, wobei der Klebstoff ein druckempfindlicher Klebstoff ist.

8. Vorrichtung nach einem der vorhergehenden Ansprüche, wobei der Klebstoff unter den Polyacrylaten, den Polyisobutylenen, den Siliconen und den Gemischen dieser Klebstoffe ausgewählt wird.

9. Vorrichtung nach einem der vorhergehenden Ansprüche, wobei der Klebstoff 10 bis 98 Gew.-% und vorzugsweise 25 bis 85 Gew.-% der klebefähigen Matrixschicht ausmacht.

10. Vorrichtung nach einem der vorhergehenden Ansprüche, wobei der Beschichtungsträger ein Polymer umfasst, das unter Polyurethan, Poly(ethylen-co-vinylacetat), Polyethylen, Polyvinylchlorid und einem Gemisch dieser Polymere ausgewählt wird.

11. Vorrichtung nach einem der vorhergehenden Ansprüche, wobei die Menge jedes Absorptionsförderers im Bereich von 1 bis 30 Gew.-% und vorzugsweise 2,5 bis 15 Gew.-% der klebefähigen Matrixschicht liegt.

12. Vorrichtung nach Anspruch 5, wobei der Harnstoff und die Milchsäure jeweils unabhängig 2 bis 6 Gew.-% der klebefähigen Matrixschicht ausmachen.

13. Vorrichtung nach Anspruch 5, wobei der Harnstoff und das N-Acetylcystein jeweils unabhängig 2 bis 10 Gew.-% der klebefähigen Matrixschicht ausmachen.

14. Vorrichtung nach Anspruch 6, wobei das Exaltolid 10 bis 20 Gew.-% der Matrixklebeschicht ausmacht.

15. Vorrichtung nach einem der vorhergehenden Ansprüche, die außerdem einen ablösbaren äußeren Überzug umfasst, der auf der klebefähigen Matrixschicht angeordnet ist.

16. Verfahren zur Herstellung einer Vorrichtung nach einem der Ansprüche 1 bis 15, das die folgenden Schritte umfasst:
i) Herstellen eines Gemischs, das (1) den Klebstoff, (2) Exaltolid oder mindestens zwei Absorptionsförderer, die unter Harnstoff, Milchsäure und N-Acetylcystein ausgewählt werden, und (3) Amorolfin oder eines seiner Derivate in einem Lösemittel umfasst, wobei der Klebstoff mit dem Lösemittel mischbar ist;
ii) Beschichten des Trägers mit dem erhaltenen Gemisch; und
iii) Verdampfen des Lösemittels.

17. Verfahren nach Anspruch 16, wobei der Klebstoff ein Polyacrylat ist.

18. Verfahren nach Anspruch 16, wobei der Klebstoff ein Silicon ist.

19. Verfahren nach Anspruch 17, wobei das Lösemittel aus Ethanol besteht.

20. Verfahren nach Anspruch 18, wobei das Lösemittel aus Dichlormethan besteht.

21. Verfahren nach einem der Ansprüche 16 bis 20, wobei das Amorolfin oder seine Derivate in dem in i) erhaltenen Gemisch solubilisiert ist / sind.

22. Verfahren nach einem der Ansprüche 16 bis 20, wobei das Amorolfin oder seine Derivate in dem in i) erhaltene Gemisch dispergiert ist/ sind.

23. Verfahren nach einem der Ansprüche 16 bis 22, wobei das in i) erhaltene Gemisch außerdem ein Co-Lösemittel umfasst, das dafür geeignet ist, das Amorolfin oder seine Derivate und/oder den oder die Absorptionsförderer in dem Gemisch zu solubilisieren.

24. Verfahren nach Anspruch 23, wobei das Co-Lösemittel aus Isopropanol besteht.

25. Verfahren nach einem der Ansprüche 26 bis 24, wobei das Lösemittel und gegebenenfalls das Co-Lösemittel 2 bis 90 Gew.-% des in Schritt i) erhaltenen Gemischs ausmachen.

26. Verfahren nach Anspruch 16, das außerdem einen Schritt iv) umfasst, in dem der ablösbare äußere Überzug aufgebracht wird.

27. Verwendung von Amorolfin oder seiner Derivate für die Herstellung eines Arzneimittels, das für die Behandlung der Onychomykose vorgesehen ist, wobei das Arzneimittel in Form einer transungualen Vorrichtung vorliegt, die wie in einem der Ansprüche 1 bis 15 definiert ist.

28. Verwendung von Amorolfin oder seiner Derivate für die Herstellung eines Arzneimittels, das für die Behandlung der Onychomykose vorgesehen ist, wobei das Arzneimittel in Form einer transungualen Vorrichtung vorliegt, die durch das Verfahren nach einem der Ansprüche 16 bis 26 erhalten wird.
